# EUROPEAN PATENT SPECIFICATION

(11) **EP 0 227 356 B1**
(45) Date of publication and mention of the grant of the patent: **16.08.1995**
(21) Application number: 86309456.1
(22) Date of filing: 04.12.1986
(51) Int. Cl.: A61K 31/485, A61K 31/52

(54) **The use of depogen in the treatment of restricted blood circulation**
Verwendung von Depogen zur Behandlung von eingeschränktem Blutkreislauf
Utilisation de dépogène pour le traitement de la circulation réduite du sang

(30) Priority: 04.12.1985 HU 463985
(43) Date of publication of application: 01.07.1987
(73) Proprietor: CHINOIN Gyogyszer és Vegyészeti Termékek Gyára RT., H-1045 Budapest IV (HU)
(72) Inventor: Szentmiklosi, Peter, H-1064 Budapest (HU); Hermecz, Istvan, H-1056 Budapest (HU); Meszaros, Zoltan, H-1113 Budapest (HU); Tardos, Laszlo, H-1111 Budapest (HU); Marton, Jeno, H-1035 Budapest (HU); Vasvari, Lelle, H-1122 Budapest (HU); Horvarth, Agnes, H-1021 Budapest (HU); Marmarosi, Katalin, H-2051 Biatorbagy (HU)
(74) Representative: Skailes, Humphrey John

(56) References cited:
- DE-A- 1 492 203
- FR-A- 2 202 686
- FR-A- 2 279 394
- FR-M- 4 135
- GB-A- 1 129 134
- GB-A- 1 417 323
- US-A- 4 364 922
- US-A- 4 383 997
- THER.HUNG., vol. 15, no. 4, pages 147-150, 1967, Budapest, HU F. SOLTI et al: "The effect of No-Spa on peripheral circulation"
- J.E.F. REYNOLDS (Ed.), Martindale, The Extra Pharmacopoeia, 28th edition, 1982, pages 341-349, 1059 The Pharmaceutical Press, London, GB
- WIEN-ZEIT. INN.MED., vol. 53, no. 10, 1972, pages 539-543, Wien, AT; P. BAUMGARTL et al.: "Kreislaufwirkungen von Drotaverin bei Gesunden mit und ohne Isoproterenolstimulation"
- ACTA PHARMACEUTICA HUNGARICA, vol. 49, 1979, pages 50-53, Budapest, HU; P. SZENTMIKLOSI et al.: "Pratical significance of drug-equivalence in preclinical research"

## Description

The invention relates to the use of a medicament for the treatment of pathological conditions caused by the restricted microcirculation of the blood by using an appropriate dose of a pharmaceutical composition containing 1-(3ʹ,4ʹ-diethoxybenzyl)-6,7-diethoxy-3,4-dihydroisoquinolinium-theophylline-7-acetate (Depogen®) as active ingredient.

It is known that Depogen® (US-PS 4,035,366) has a peripheral vasodilating effect and decreases the extremital vascular resistance (Acta Pharmaceutica Hung. 49, 50-53 (1979)).

It is also known that the arteriosclerotic diseases - mainly in the well developed industrial countries - are widespread and are prominent causes of death. In case of people suffering from the above clinical symptom - patients suffering from occlusive vascular process of the lower leg or from vascular encephalopathy - the wall of the resistant arteries will be thicker, the number of the vegetative receptors decreases and the flexibility and the blood permeability of the arteries deteriorate.

In the treatment of such patients, peripheral vasodilating agents have hitherto been used. When the sclerotic arteries are, however, in even worse condition, they are less dilatable and the possibility of using vasodilating agents is limited (Martindale: The Extra Pharmacopeia XXVIIIth Ed. (1982) London, P. 1614).

There is also known a disease of probable genetic, hereditary origin where the transformation of the erythrocytes into sickle cells or their loss of flexibility are manifested in significant clinical symptoms (Sickle-cell anaemia). (Dorlands Illustrated Medical Dictionary 25, 860 Ed. Sanders (1974).) This illness again cannot be treated by the usual drugs (Klinische Pharmakologie und Pharmakotherapie Künmerle, Garett, Tzyzi Urban, Schwarzenberg München, 1976, p. 911).

It is also known that the 3,7-dihydro-3,7-dimethyl-1-(5-oxohexyl)-1H-purine-2,6-dione (pentoxiphyllin; (Trental^{R})) improves some rheological parameters of the blood, particularly the plasticity of the erythrocytes, and thus it can be used for treating clinical conditions where the deterioration of the plasticity of the erythrocytes causes poor microcirculation and secondary tissue hypoxia, with good results (Angiology 36, 4, 226-234 (1985)).

By improving their filtrability, the erythrocytes may pass through the capillaries and thus the oxygen supply of the tissues improves significantly.

By influencing the haemorheological properties of the blood, namely increasing its fluidity, many pathological conditions may be influenced favourably (e.g. decreasing the danger of thrombosis after operations, prophylaxis of myocardial infarcts, slowly healing wounds caused by poor blood supply, ulcers, embolism, cerebral infarcts, shock, chronic vascular diseases, claudication, senile cerebral circulatory disturbances, diabetes) and this is of great therapeutical importance both systemically and locally.

It has been found that Depogen® when administered at a suitable dose possesses very favourable haemorheological properties. It increases the filtrability of the pathological human erythrocytes significantly, as proved by both in vitro and in vivo tests.

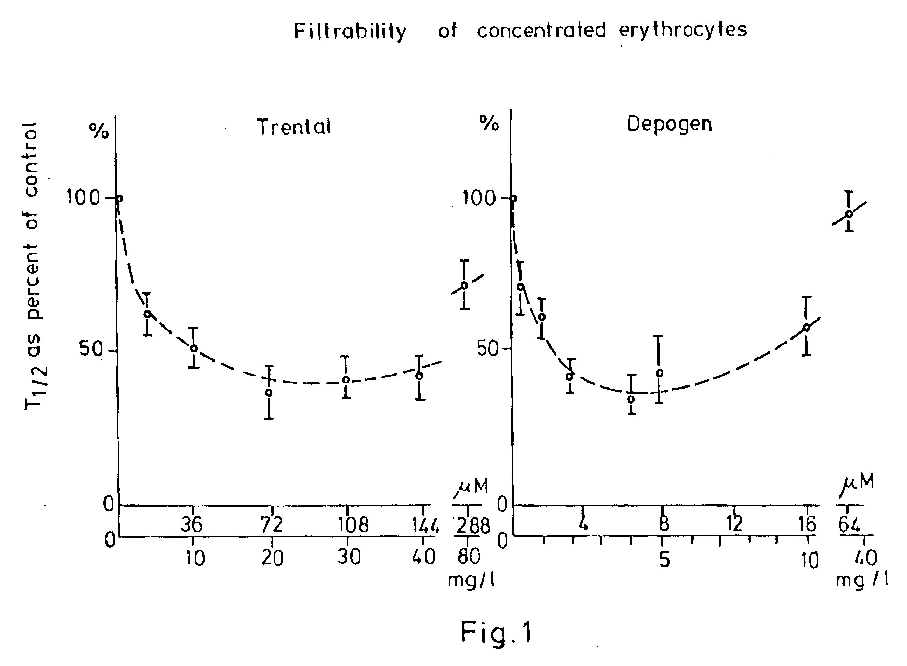

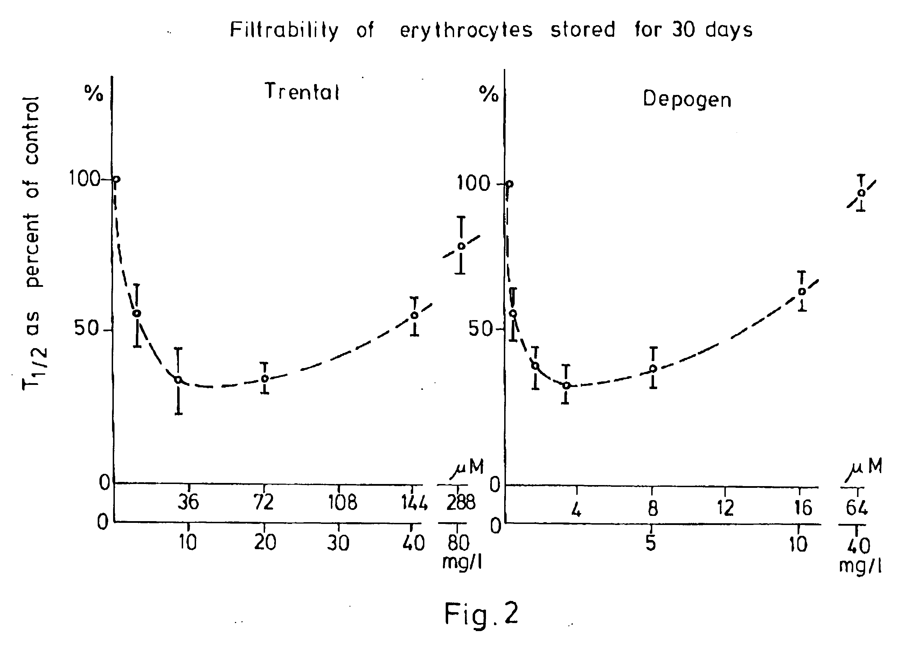

For the examination of the in vitro filtrability of the erythrocytes the method of Teitel (Nouv. Rev. Franc. Haemotol. 7, 195 (1967)) was used where the filtration rate of concentrated erythrocyte suspension was measured. In the test fresh, normal erythrocytes, fresh erythrocytes and erythrocytes stored in CPO blood-conserving solution for 30 days at +4°C were used. The results are given in Figures 1 and 2 (the filtrability of the concentrated erythrocytes and that of the erythrocytes stored for 30 days were given as the % of the filtrability of the control fresh, normal erythrocytes).

From Figures 1 and 2 it may be seen that both Trental^{R} and Depogen® increase the filtrability of the erythrocytes significantly, i.e. decrease the T_{½}-value characteristic for the perfusion (the time which is needed for the perfusion of the half amount of the erythrocytes). The effect depends in case of both drugs on the dose. The optimal effect may be achieved with Depogen® over a lower concentration range (0.5 to 1.5 »mol) than in case of Trental® (5 to 10 »moles). Depogen® also exerts the same effect as Trental® at a 10 to 25-times lower concentration, as shown by the concentration necessary for the half-maximal effect (ID₅₀, Table I).

**Table I**

| | Trental® ID₅₀ »mol | Depogen® ID₅₀ »mol | Rel. activity Trental/Depogen |
|---|---|---|---|
| concentrated erythrocytes | 36 | 2.2 | 16.3 |
| erythrocytes stored for 30 days | 15.8 | 0.5 | 31.6 |

Clinical examinations carried out on patients suffering from lower leg and cerebral occlusive vascular diseases gave the following results: 41 patients were treated for 10 days with a dose of 450 mg/day Depogen® or Trental® orally (together with 5 healthy controls). The favourable effect of Depogen® in cerebral vascular disease has been found and the symptoms have decreased. The results of the examinations carried out with EEG and ECHO are given in Tables 2 and 3. In case of lower leg obliterations the two drugs have the same effect.

The filtrability of the erythrocytes is much lower (T_{½} average = 18.15 min) as compared to the healthy control (T_{½} average = 8.7 min; (T_{½} normal = 6.5 to 10.5 min) before treatment. During a 10 days Depogen® treatment the filtrability of the erythrocytes has been normalized (T_{½} average = 9.53 min) in case of the majority of the patients, independently of whether the patient had a cerebral or lower leg disease. This effect was much stronger than in case of patients treated with Trental although the treatment was also in this case successful (T_{½} average = 12.1 min). The improvement in the filtrability of the erythrocytes correlated to the decrease of the clinical symptoms.

The filtrability of the erythrocytes was unchanged in the healthy controls (T_{½} average = 8.7 min).

It has been surprisingly found that when the decrease of the filtrability of the erythrocytes was stronger, the therapeutic effect was also stronger.

In view of the above, the use according to the invention is also suitable for determining the dose to be used in the treatment, for preindication of the necessity of the therapy or of the efficiency of the treatment in view of the filtrability of the erythrocytes determined from the blood of the patient.

In the treatment of restricted circulation, a daily dose is preferably used which is necessary for achieving a drug blood concentration of 0.5 to 3»g/ml calculated on the 1-(3ʹ,4ʹ-diethoxybenzyl)-6,7-diethoxy-3,4-dihydrioisoquinoline.

We have found that such blood concentrations may be achieved at a daily dose of 400-800 mg, preferably using slow release formulations (e.g. 400 my retard tablets). In general treatment should be maintained for a minimum of 10 days. These doses are generally higher and the duration of treatment shorter (or effective treatment achieved more quickly) than suggested in the above references.

In the treatment the drug may be used in form of capsules, tablets, retard tablets, injections, solutions or syrups in bolus or infusion.

Chemical composition of the components given in the following examples by trade names:
- CMC-Na -: Carboxymethyl cellulose NapH.Hg. VI.
- Tween® 85 -: Sorboxaethenum trioleinicum Merck
- Tween® 20 -: Sorboxaethenum laurinicum Ph.Hg. VI.
- Aerosil® 300 -: Acidum silicicum colloidale Ph.Hg.VI.
- Kolloidon V.A. 64 -: polyvinylpyrrolidone Ph-Hg. VI.
- Amylum mays -: corn starch
- Cutine H.R. -: hardened castor oil (Oleum ricini hydrogenatum)
Solutio conservans pH.Hg.VI (Nipasol + Nipagin)
- Softisan® - 378 -: mixture of triglycerides of saturated fatty acids of natural origin
- Avicel® -: F.M.C. Corp. Philadelphia USA.

### Example 1 - Depogen retard tablet (100 units)

| | |
|---|---|
| Depogen® | 40.00 g |
| CMC-Na | 8.50 g |
| Gypsum | 52.90 g |
| Stearic acid | 4.80 g |
| Tween® 85 | 0.15 g |
| Tween® 20 | 0.10 g |
| Mg-stearate | 1.00 g |
| Talcum | 2.00 g |

### Example 2 - Depogen capsule

| | |
|---|---|
| Depogen® | 150 mg |
| Aerosil® 300 | 3 mg |
| Kollidon V.A. 64 | 12 mg |
| Amylum mayidis | 54 mg |
| Avicel® R 581 | 66 mg |
| Cutine H.R. | 3 mg |
| Mg-stearate | 3 mg |
| Talcum | 9 mg |

### Example 3 - Syrup

| | |
|---|---|
| Depogen® | 1.00 g |
| Cacao powder | 10.00 g |
| Saccharosum | 45.00 g |
| Glycerinum | 45.00 g |
| Natrium chloratum | 0.05 g |
| Solutio conservans | 0.50 g |
| Spiritus dilutus | 2.00 g |
| Aqua destillata ad. | 100.00 g |

### Example 4 - Drops

| | |
|---|---|
| Depogen® | 40.00 mg |
| Spir. conc. | 0.04 ml |
| Aqua bidestillata ad. | 1.00 ml |

### Example 5 - Suppository

| | |
|---|---|
| Depogen® | 0.15 g |
| Witepsol® H 32 | 2.57 g |
| Tagat® R-1 | 0.14 g |
| Softisan® 370 | 0.14 g |

### Example 6 - Tablets (100 units)

| | |
|---|---|
| Depogen*® | 40 g |
| Eudragit® 100-55 | 8 g |
| NaOH | 0.08 g |
| Aerosil® R972 | 0.3 g |
| Mg stearate ad. | 50 g |

| | |
|---|---|
| *or stoichiometric equivalent of its monohydrate. | |

**Table 2**

| The effect of Trental® and Depogen® as determined by EEG examination | | | | |
|---|---|---|---|---|
| | unchanged | deteriorated | improved | total |
| TRENTAL® | 5 /55.5%/ | 0 | 4 /44.5%/ | 9 |
| DEPOGEN® | 1 /12.5%/ | 0 | 7 /87.5%/ | 8 |

**Table 3**

| The effect of Trental® and Depogen® as determined by ECHO examination | | | | |
|---|---|---|---|---|
| | unchanged | deteriorated | improved | total |
| TRENTAL® | 9 /100%/ | 0 | 0 | 9 |
| DEPOGEN® | 7 /87.5%/ | 0 | 1 /12.5%/ | 8 |

## Claims

1. The use of 1-(3',4'-diethoxybenzyl)-6,7-diethoxy-3,4-dihydroisoquinolinium-theophylline-7-acetate (Depogen®) for the manufacture of a medicament for increasing the filtrability of the pathological erythrocytes in conditions resulting from restricted circulation of the blood in the smaller blood vessels.

2. Use according to claim 1 which comprises presenting the drug in the form of capsules, tablets, retard tablets, injections, solutions or syrups.

3. Use according to claim 1 or claim 2 in which said condition is an occlusive vascular disease.

4. Use according to any preceding claim in which the medicament is in dosage unit form comprising 400-800 mg Depogen®.

## Patentansprüche

1. Verwendung von 1-(3',4'-Diethoxybenzyl)-6,7-diethoxy-3,4-dihydroisochinolin-Theophyllin-7-acetat (Depogen®) zur Herstellung eines Medikaments zur Verbesserung der Filtrierbarkeit von pathologischen Erythrocyten unter den Bedingungen einer eingeschränkten Blutzirkulation in den kleineren Blutgefäßen.

2. Verwendung gemäß Anspruch 1, dadurch gekennzeichnet, daß das Medikament in Form von Kapseln, Tabletten, Retard-Tabletten, Injektionen, Lösungen oder Sirupen angewendet wird.

3. Verwendung gemäß Anspruch 1 oder 2, dadurch gekennzeichnet, daß das Medikament unter der Bedingung einer okklusiven Gefäßerkrankung verwendet wird.

4. Verwendung gemäß einem der vorhergehenden Ansprüche, dadurch gekennzeichnet, daß das Medikament in Form von Dosierungseinheiten mit 400 - 800 mg Depogen® verwendet wird.

## Revendications

1. Application du du 1-(3',4'-diéthoxybenzyl)-6,7-diéthoxy-3, 4-dihydroisoquinoléinium-théophylline-7-acétate (Depogen®) pour la préparation d'un médicament pour augmenter la filtrabilité des érythrocytes pathologiques dans les états résultant de la restriction de la circulation du sang dans les petits vaisseaux sanguins.

2. Application selon la revendication 1 dans laquelle on présente le médicament sous forme de gélules, de comprimés, de comprimés retard, d'injections, de solutions ou de sirops.

3. Application selon la revendication 1 ou la revendication 2 dans laquelle ledit état est une maladie vasculaire occlusive.

4. Application selon l'une quelconque des revendications précédentes dans laquelle le médicament est sous une forme posologique unitaire comprenant 400-800 mg de Depogen®.
